# EUROPEAN PATENT SPECIFICATION

(11) **EP 3 888 661 B1**
(45) Date of publication and mention of the grant of the patent: **17.12.2025**
(21) Application number: 20167199.7
(22) Date of filing: 31.03.2020
(51) Int. Cl.: A61K 31/702, A61K 45/06, A61P 31/14

(54) **COMPOSITIONS COMPRISING 2 -FUCOSYLLACTOSE TO PREVENT VIRAL INFECTIONS**
ZUSAMMENSETZUNGEN MIT 2-FUCOSYLLAKTOSE ZUR VERHINDERUNG VON VIRUSINFEKTIONEN
COMPOSITIONS COMPRENANT DU 2-FUCOSYLLACTOSE POUR LA PRÉVENTION DES INFECTIONS VIRALES

(43) Date of publication of application: 06.10.2021
(73) Proprietor: FrieslandCampina Nederland B.V., 3818 LE Amersfoort (NL)
(72) Inventor: NAUTA, Arjen, 6708 WH Wageningen (NL)
(74) Representative: FrieslandCampina IP Department

(56) References cited:
- WO-A1-2011/008087
- WO-A1-2015/071391
- WO-A1-2016/014473
- WO-A1-2020/013684
- US-A1- 2015 031 645
- GERALYN DUSKA-MCEWEN ET AL: "Human Milk Oligosaccharides Enhance Innate Immunity to Respiratory Syncytial Virus and Influenza in Vitro", FOOD AND NUTRITION SCIENCES, vol. 5, 1 January 2014 (2014-01-01), pages 1387 - 1398, XP055480727, ISSN: 2157-944X, DOI: 10.4236/fns.2014.514151
- SPRENGER N ET AL: "HMO stimulated bifidobacteria contribute to risk reduction for lower respiratory tract illnesses with a 2-HMO containing infant formula through protective effects on mucosal barrier function", 1 May 2019, JOURNAL OF PEDIATRIC GASTROENTEROLOGY AND NUTRITION 20190501 LIPPINCOTT WILLIAMS AND WILKINS NLD, VOL. 68, NR. SUPPLEMENT 1, PAGE(S) 1232 CONF 20190605 TO 20190608 GLASGOW- 52ND ANNUAL MEETING OF THE EUROPEAN SOCIETY FOR PAEDIATRIC GASTROENTEROLOGY H, ISSN: 1536-4801, XP055731499
- KRIST HELEN ANTUNES ET AL: "Microbiota-derived acetate protects against respiratory syncytial virus infection through a GPR43-type 1 interferon response", NATURE COMMUNICATIONS, vol. 10, no. 1, 22 July 2019 (2019-07-22), XP055731387, DOI: 10.1038/s41467-019-11152-6

## Description

### FIELD OF THE INVENTION

The invention disclosed herein relates to a composition comprising 2'-fucosyllactose (2'-FL) for use in the prevention against and / or the treatment of RNA-virus infection by increasing the production of short chain fatty acids (SCFA) in the distal colon; the composition is a nutritional composition and wherein the virus is a Group IV type virus as defined in the Baltimore Classification of viruses.

### BACKGROUND

WO2016014473 discloses a nutritional composition comprising 2'-fucosyllactose (2'FL) for use in the treatment of respiratory virus infections and more particularly respiratory syncytial virus (RSV) infection. The composition further comprises probiotics or a prebiotic dietary fiber such as inulin.

WO2011008087 discloses a nutritional composition comprising 2'-fucosyllactose (2'FL) for use in the treatment of viral infections, more preferably viral infections caused by the influenza virus, rotavirus, respiratory syncytial virus or HIV.

US20150031645 discloses a nutritional composition comprising 2'-fucosyllactose for use in the treatment of a respiratory viral infection such as influenza virus or RSV.

Geralyn Duska-McEwen et al, FOOD AND NUTRITION SCIENCES, vol. 5, 1 January 2014 (2014-01-01), pages 1387-1398, DOI: 10.4236/fns.2014.514151 teaches that 2'-FL decreases the RSV viral load in respiratory epithelial cell lines.

N. Sprenger et al, (2019-05-01), Journal Of Pediatric Gastroenterology And Nutrition Lippincott Williams and Wilkins NLD, vol. 68, nr. Supplement 1, page(s) 1232 CONF 20190605 TO 20190608 GLASGOW- 52ND Annual Meeting Of The European Society For Paediatric Gastroenterology H, ISSN: 1536-4801 teaches that human milk oligosaccharides stimulated bifidobacteria contribute to risk reduction for lower respiratory illnesses with 2'-FL containing infant formula through protective effects on mucosal barrier function.

WO2015071391 discloses a nutritional composition comprising 2'-fucosyllactose for use in the treatment of an influenza infection.

WO2020013684 discloses nutritional composition comprising 2'-fucosyllactose for use in the treatment of a rotavirus infection.

A viral disease (or viral infection, or virus infectious disease) occurs when an organism's body is invaded by pathogenic viruses, and infectious virus particles (virions) attach to and enter susceptible cells.

Virus classification is the process of naming viruses and placing them into a taxonomic system. Viruses are mainly classified by phenotypic characteristics, such as morphology, nucleic acid type, mode of replication, host organisms, and the type of disease they cause. The formal taxonomic classification of viruses is the responsibility of the International Committee on Taxonomy of Viruses (ICTV) system. In addition, the Baltimore classification system is used to place viruses into one of seven groups based on their manner of mRNA synthesis. Baltimore classification (D. Baltimore Bacteriological Reviews 1971 Vol 35 No 3 pp 235 241) is a classification system that places viruses into one of seven groups depending on a combination of their nucleic acid (DNA or RNA), strandedness (single-stranded (ss) or doublestranded (ds)), sense, and method of replication. Classifying viruses according to Baltimore Classification means that those in a given category will all behave in a similar fashion, offering some indication of how to proceed with further research. Viruses can be placed in one of the seven Baltimore groups (designated by Roman numerals): [ http://www.web-books.com/ under MoBio/Free/Ch1E2.htm ]:
I: dsDNA viruses (e.g. Adenoviruses, Herpesviruses, Poxviruses)
II: ssDNA viruses (+ strand or "sense") DNA (e.g. Parvoviruses)
III: dsRNA viruses (e.g. Reoviruses, Rotaviruses)
IV: (+)ssRNA viruses (+ strand or sense) RNA (e.g. Picornaviruses, Togaviruses)
V: (-)ssRNA viruses (- strand or antisense) RNA (e.g. Orthomyxoviruses, Rhabdoviruses)
VI: ssRNA-RT viruses (+ strand or sense) RNA with DNA intermediate in life-cycle (e.g. Retroviruses)
VII: dsDNA-RT viruses DNA with RNA intermediate in life-cycle (e.g. Hepadnaviruses)

An RNA virus is a virus in which the genetic information is stored in the form of RNA (as opposed to DNA). This can be + or - strand (sense or antisense) RNA. A human virus is a virus which is able to infect a human. A respiratory virus is a virus that can enter and invade the respiratory system, i.e. the system from the nose to the lungs, in other words, the system allowing to breathe-in oxygen and breath-out carbon dioxide. A human respiratory virus is a respiratory virus which is able to infect a human. Respiratory syncytial virus (RSV, or RS virus) is the cause of RSV infection and is a major cause of morbidity and mortality in infants < 2 years-old. RSV is a Baltimore Group V type virus, a single strand RNA virus, and is a member of the *Paramyxovidae* virus family. RSV is also a major cause of respiratory illness in the elderly. A virus may, such as a respiratory virus e.g. RSV, may be transferred from one person to another by direct physical contact, or via coughing or sneezing. Aerosols produced during coughing or sneezing may be inhaled by another person thereby allowing virus particles to move from one person to another. Approved vaccines do not yet exist, and whilst childhood infection induces partial immunity, individuals remain susceptible to RSV reinfection life-long.

Antunes *et al* have shown that feeding mice a high-fibre (HF) diet protects mice from RSV infections. This effect correlated with the production of acetate in the gut. Oral administration of acetate, mediated interferon-β (IFN-β) response by increasing expression of interferon-stimulated genes in the lung. These effects were associated with reduction of viral load and pulmonary inflammation in RSV-infected mice. Activation of Gpr43 in pulmonary epithelial cells reduced virus-induced cytotoxicity and promoted antiviral effects through IFN-β response. The findings reveal antiviral effects of acetate involving IFN-β in lung epithelial cells and engagement of GPR43 and IFNAR. A HF diet protected against RSV disease by reducing viral load. High Fibre diet protected mice from RSV-induced weight loss and also reduced lung viral load. HF diet significantly reduced the numbers of total cells in the bronchoalveolar lavage fluid (BALF), mainly attributed to reduction of macrophages. HF diet also reduced lung histological inflammatory score (Antunes et al 2019 Nat. Commun. 10, 3273 (2019) | https://doi.org/10.1038/s41467-019-11152-6).

Antunes et al thus have shown that higher acetate levels in the gut, protects mice against RSV-induced disease.

There is thus a need for ways to prevent or at least reduce the symptoms of viral infections. In particular to *Paramyxoviridae*, to RNA viruses, to single strand RNA viruses and/or to RNA viruses of the Baltimore Group IV or V, in particular to the Respiratory syncytial virus. There is also a desire to reduce or minimize the need to use antiviral treatments, both in view of costs and in view of health comfort.

In addition, it is desired that such a prevention or reduction is compatible with a normal and/or healthy diet. Preferably without negative effects on taste (of food) and/or mouth feeling. Other desired properties or effects of the prevention or reduction of symptoms of viral infection include, but are not limited to, maintaining other signs of general health in the subject (*e.g*. maintaining a normal blood pressure, maintaining a healthy bowel movement, normal defecation, etc.), ease of implementation, ease of preparation, increasing the diversity of microbiota, and commercial availability of the compounds, combinations, and/or compositions used therein.

It is an objective of the present invention to provide a composition that better addresses at least one of the aforementioned desires.

### SUMMARY OF THE INVENTION

The invention as described herein is based on the finding that a specific dietary fiber i.e. 2'-fucosyllactose (2'-FL) can be administered, preferably orally, to a human subject to achieve at least one of the above desires. In particular, it was found that a composition comprising 2'-FL substantially increases the level of acetate in the distal part of the colon in human subjects, consequently serum acetate levels as produced after the portal vein (and thus not passing the liver). Therefore, the invention relates to a composition comprising 2'-fucosyllactose (2'-FL) for use in the prevention against and / or the treatment of RNA-virus infection by increasing the production of short chain fatty acids (SCFA) in the distal colon; the composition is a nutritional composition wherein the virus is a Group IV type virus as defined in the Baltimore Classification of viruses.

### DETAILED DESCRIPTION OF THE INVENTION

Note that the references to the methods of treatment by therapy of this description are to be interpreted as references to compounds, pharmaceutical compositions and medicaments of the present invention for use in those methods.

The term "treatment", in relation a given disease or disorder, includes, but is not limited to, inhibiting the disease or disorder, for example, arresting the development of the disease or disorder; relieving the disease or disorder, for example, causing regression of the disease or disorder; or relieving a condition caused by, or resulting from, the disease or disorder, for example, relieving, preventing or treating symptoms of the disease or disorder.

The term "prevention" in relation to a given disease or disorder means preventing the onset of disease development if none had occurred, preventing the disease or disorder from occurring in a subject that may not yet have been diagnosed as having the disorder or disease, preventing further disease/disorder development if already present, and / or reducing the symptoms of a disease/disorder as compared to a subject not taking the treatment of the invention.

The term "disease", which is used herein as synonym of "disorder", encompasses a viral infection.

As used herein, "prevention of a virus infection" is defined as ameliorating the risk of suffering from a virus infection. Suffering from a respiratory virus infection in a young child may for example be measured by the number of GP visits for cough and/or wheeze during the first 12 or 24 months of a child's life.

2'-Fucosyllactose (2'-FL or 2'FL) is an oligosaccharide, more precisely, a fucosylated, neutral trisaccharide composed of L-fucose, D-galactose, and D-glucose units, linked Fuc(α1-2)Gal(β1-4)Glc; CAS Nr 41263-94-9. It is the most prevalent human milk oligosaccharide (HMO) naturally present in human breast milk, making up about 30% of all of HMOs, at least in so called secretor mothers (i.e. FUT2 positive). HMOs are non-digestible carbohydrates and are the third most abundant component in human milk after lactose and fat. More than 200 different oligosaccharides have currently been identified in human milk. It has been suggested in clinical trials that 2'-FL plays a key role in protecting and promoting the health of newborn infants, particularly in respect to the immune system. It has been shown that the addition of 2'-FL to infant formula is safe and well-tolerated. In addition, 2'-FL is safe and well-tolerated for all other age groups, especially for adults.

HMOs can be obtained using methods known to those of skill in the art. For example, HMOs can be purified from human milk. Individual HMOs can be further separated using methods known in the art such as capillary electrophoresis, HPLC (*e.g*., high-performance anion-exchange chromatography with pulsed amperometric detection; HPAEC-PAD), and thin layer chromatography. See, *e.g*., U.S. Patent Application No. 2009/0098240. Alternately, enzymatic methods can be used to synthesize HMOs. Another method to manufacture HMO's is via biosynthesis in engineered bacteria. For example, a method of preparing 2'-FL is disclosed in WO 2012/112777. Alternatively, 2'-FL is commercially available e.g. from FrieslandCampina, or others.

An amount of a compound such as 2'-FL, in an composition, expressed in "a number of grams (or milligrams) per daily serving" as used herein means that the amount of the compound in the composition is such that when administering the recommended daily serving (i.e. daily dosage) of the composition to a subject, the subject will be administered with the number of grams of the compound. So, if the recommended daily dosage is 100 gram of composition divided over 2 portions, then a single serving consists of 50 gram of composition; a daily serving consists of 2 of such single servings. If the daily serving of 2' FL is 2 grams of 2'FL, then the recommended total amount of composition is such that the subject is administered 2 grams of 2'FL. This may be in one or more portions. For example, if the daily serving is 2 grams of 2'FL and the composition comprises 2 wt% of 2'FL , then the recommended dosage is 100 gram of composition. These 100 gram of composition may be divided over one or more portions e.g. 1x 100 g or 2x50 g or 4 x 25 g. Each such portion may be referred to "single serving" or single serving size".

As used herein, dietary fibers refers to carbohydrates which are neither digested nor absorbed by humans but are utilized by gut microbes. They can be isolated from plants or synthesized from sugars and are comprising at least 2 monosaccharide units (i.e. degree of polymerization DP≥ 2, preferably DP≥3). Their fermentation in the gastrointestinal tract impacts the composition of bacterial communities as well as microbial metabolic activities, including the production of fermentative end products in the gut of the host. Some dietary fibers can also be classified as prebiotic. A prebiotic is defined as a non-digestible food ingredient that promotes the growth of beneficial microorganisms in the intestines of the host. Consumption of prebiotics result in specific changes in the composition and/or activity of the gastrointestinal microbiota, thus conferring benefit(s) upon host health.

Sources of prebiotic dietary fiber can be selected from one or more of the group consisting of beta glucan, Fructo-oligosaccharides (FOS) including inulin, Galacto-oligosaccharides (GOS), guar gum, resistant starches (RS), maltodextrin, xylooligosaccharides and arabinooligosaccharides. Preferably, the prebiotic dietary fiber is selected from one or more of the group consisting of resistant starch, FOS and GOS. Prebiotic dietary fibers are claimed to have several health effects such as effect on gut microbacteria, metabolite production such as short chain fatty acids (SCFAs) that can have many positive outcomes for the host, effects on mineral absorption, effects on protein fermentation, changes in bacterial pathogenic populations, effects on allergy risk, effects on gut barrier permeability, and effect on immune system defense. See for example Health Effects and Sources of Prebiotic Dietary Fiber by Calson et al 2018, Curr Dev Nutr 2018;2:nzy005 doi: https://doi.org/10.1093/cdn/nzy005.

Fructooligosaccharides (FOS), also sometimes called oligofructose or oligofructan, are oligosaccharide fructans. They may be obtained using inulin degradation or transfructosylation processes, using methods known in the art. FOS can be produced by degradation of inulin, or polyfructose, a polymer of D-fructose residues linked by β(2→1) bonds with a terminal α(1→2) linked D-glucose. The degree of polymerization of inulin ranges from 10 to 60. Inulin can be degraded enzymatically or chemically to a mixture of oligosaccharides with the general structure Glu-Fruₙ (abbrev. GFn), with n ranging from 1 to 7. The second class of FOS is prepared by the transfructosylation action of a β-fructosidase of an *Aspergillus* e.g. *Aspergillus niger* on sucrose. The resulting mixture has the general formula of GFm, with m ranging from 1 to 5.

The term "GOS" as used herein, stands for galacto-oligosaccharides (GOS), which generally comprise a chain of galactose units and a glucose unit at the reducing end. GOS arise through consecutive trans-galactosylation reactions catalyzed by a beta-galactosidase (enzyme class EC.3.2.1.23). Beta-Galactosidase enzymes are produced in many microorganisms such as *Bacillus circulans, Aspergillus oryzae, Kluyveromyces marxianus, Kluyveromyces fragilis, Sporobolomyces singularis,* and *Lactobacillus fermentum.* Beta-galactosidases differ in their three-dimensional structures, resulting in stereo- and regioselectivity of the glycosidic bonds that are formed during the trans-galactosylation reactions. For example, typically a fungal beta-galactosidase derived from Aspergillus predominantly produces β1-6 bonds (thus resulting in a GOS preparation that predominantly comprises β1-6 bonds, which may be referred to as "6'-GOS"), while a bacterial beta-galactosidase derived from Bacillus predominantly produce β1-4 bonds (resulting in a GOS preparation that predominantly comprises β1-4 bonds, which may also be referred to as "4'-GOS"). Moreover, beta-galactosidase produced by *B. circulans* possesses particularly strong trans-galactosylation activity, and is often used to synthesize GOS. More recently, a beta-galactosidase derived from *Cryptococcus terrestris* (recently renamed *Papiliotrema terrestris*) was used in GOS synthesis, as described in EP3399032 and WO 2019/002304. As such, GOS is well-known in the art.

Some of the GOS components exist naturally in human breast milk and colostrum. Typical GOS preparations mainly comprise di- to hexa-saccharides, although larger oligosaccharides may also occur.

Various physiological functions of GOS have been reported, including the capacity to stimulate the growth of bifidogenic bacteria in the gut, to support normal gut transit, to contribute to natural defenses and to enhance mineral absorption. GOS has received particular attention for their prebiotic effects that promote the growth of Bifidobacterium, Lactobacillus, and other enteric bacteria. Therefore, GOS is commonly used in infant formula, beverages fermented by Lactobacillus, and yogurts. Some of these foods containing GOS are certified as Food for Specified Health Uses by the Consumer Affairs Agency in Japan, and GOS is certified as generally recognized as safe (GRAS) substances by the U.S. Food and Drug Administration (GRAS Notices: GRN 233, 236, 285, 286, 334, 484, 489, 495, 518, and 569).

Preferably, GOS has a polymerization degree (DP) in a range of from 2 to 10 more preferably in a range of from 3 to 8. Suitable GOS preparations are commercially available, for example Vivinal^{®} GOS available from FrieslandCampina Nederland B.V.. In this respect, it is noted that a GOS preparation (e.g. Vivinal^{®} GOS) may also contain lactose and/or monosaccharides in addition to galacto-oligosaccharides. In relation to the invention, the amounts of GOS mentioned in the present application relate to the actual GOS content in a preparation, i.e. the actual amount of galacto-oligosaccharides, excluding lactose and excluding monosaccharides, if present.

The term "synthetic composition", which may also be referred to as "synthetic nutritional composition", as used herein refers to a composition which is artificially prepared and preferably means a composition comprising at least one compound that is produced ex vivo chemically and/or biologically, e.g. by means of chemical reaction, enzymatic reaction or recombinantly, or purified by humans. Preferably, the synthetic composition of the invention is not identical with a naturally occurring composition. The synthetic composition of the invention typically comprises one or more compounds, advantageously 2'FL, but may further include other ingredients like protein, fat, minerals or vitamins.

It must also be noted that, as used in the specification and the appended claims, the singular form "a", "an," and "the" comprise plural referents unless the context clearly indicates otherwise. For example, reference to a component in the singular is intended to comprise a plurality of components.

It will be understood that within this disclosure, any reference to a weight, weight ratio, and the like pertains to the dry matter, in particular the dry matter of the composition, unless specified otherwise.

Unless defined otherwise, all technical and scientific terms used herein generally have the same meaning as commonly understood by one of ordinary skill in the art to which this invention belongs.

The inventor surprisingly found that the administration to 2'FL to a model system of the human gut, resulted in an increase in the production of short chain fatty acids (SCFA), in particular of acetate in the distal colon (the part of the colon after the portal vein i.e. the descending colon (the left side of the colon) and the sigmoid colon). This finding makes it plausible that acetate production in the gut of a human subject consuming 2'FL increases and thereby results in an increase of the acetate levels in circulation. Anthunes et al (*vide supra*) have shown that increased acetate levels in the gut result in a protection against RNA-viral infections. Such a protection may result in lower counts of viral RNA in the subject's respiratory tract, a shorter period wherein the subject is contagious, or a shorter window of infection i.e. less symptoms and /or a shorter period wherein the virus particles may be detected in the subject. So, in a first aspect the invention relates to a composition comprising 2'-fucosyllactose (2'-FL) for use in the prevention against and / or the treatment of RNA-virus infection, preferably the prevention or treatment of a RNA-virus infection in a human. Preferably the composition is a nutritional composition. More preferably, the composition is a synthetic nutritional composition. So, in one embodiment, the invention relates to such synthetic composition for use in the prevention against and / or the treatment of RNA-virus infection by increasing the production of short chain fatty acids (SCFA) in the distal colon; the composition is a nutritional composition wherein the virus is a Group IV type virus as defined in the Baltimore Classification of viruses.

The RNA virus may be a human virus, preferably the virus is a human respiratory virus. In one embodiment the virus is a single strand RNA virus, preferably a single strand human RNA virus, more preferably a single strand human respiratory RNA virus.

In one embodiment of any of the aspects of the invention, the human subject as referred to in the various aspects and embodiments of the invention may have any age, e.g. a baby (0-12 months), toddler (1-3 years), preschool child (3-5 years), grade-schooler (5-12 years), teen (12-18 years), young adult (18-21 year) or an adult (>21 years).

In one embodiment, the composition for use in the prevention against virus infection and/or treatment of virus invention of the invention is a food product, preferably a food product selected from the group consisting of dairy product, bar, liquid product, savory snack, savory biscuit, bakery products, pasta and food supplement. More preferably, the composition is a dairy product. The synthetic composition of the invention may further comprise at least one source of fat, at least one source of protein and at least one source of carbohydrate. Generally, any source of protein, carbohydrate, or fat that is suitable for use in nutritional products is also suitable for use herein, provided that such macronutrients are also compatible with the essential elements of the nutritional composition as defined herein.

In one embodiment, the synthetic composition of the invention is an infant formula, preferably, wherein the composition is a formula for children having an age selected from the group consisting of 0-6 months, 0-12 months, 6-12 months 12-24 months, 12-36 months and 24-36 months. More preferably, wherein the composition is a formula for children having an age of 0-6 months, 0-12 months, or 12-36 months. In another embodiment the composition is an adult formula.

The terms "infant formula" or "infant nutritional product" as used herein are used interchangeably to refer to nutritional compositions that have the proper balance of macronutrients, micro-nutrients, and calories to provide sole or supplemental nourishment for and generally maintain or improve the health of infants, toddlers, or both. Infant formulas preferably comprise nutrients in accordance with the relevant infant formula guidelines for the targeted consumer or user population, an example of which would be the Infant Formula Act, 21 U.S.C. Section 350(a). Another example with guidelines for nutrients of an infant formula, in particular for a person of 0-12 months of age and for children up to 36 months old, may be found in the CODEX Alimentarius (CODEX STAN 72-1981), further referred to as the CODEX). Nutritional compositions for infants are commonly referred to as infant formula. When used as infant formula, the composition as used in the various aspects of the invention should contain the ingredients in the amounts as prescribed by the CODEX and, if needed, as prescribed by additional regulations of individual countries. An example of an ingredient list of an infant formula meeting the requirements of the EU, China and Codex can for example be found on www.frieslandcampinaingredients.com/ at app/uploads/2019/04 /PDS_ELN_ Essential^{®}-Start-IF-110.pdf.

In certain embodiments, when the nutritional powder is formulated as an infant formula, the protein component is typically present in an amount of from 5% to 35% by weight of the infant formula (i.e., the dry weight), including from 10% to 30%, from 10% to 25%, from 15% to 25%, from 20% to 30%, from 15% to 20%, and also including from 10% to 16% by weight of the infant formula (i.e., the dry weight). The carbohydrate component is typically present in an amount of from 40% to 75% by weight of the infant formula (i.e., the dry weight), including from 45% to 75%, from 45% to 70%, from 50% to 70%, from 50% to 65%, from 50% to 60%, from 60% to 75%, from 55% to 65%, and also including from 65% to 70% by weight of the infant formula (i.e., the dry weight). The fat component is typically present in an amount of from 10% to 40% by weight of the infant formula (i.e., the dry weight), including from 15% to 40%, from 20% to 35%, from 20% to 30%, from 25% to 35%, and also including from 25% to 30% by weight of the infant formula (i.e., the dry weight).

In certain embodiments, when the nutritional powder is formulated as a pediatric formula, the protein component is typically present in an amount of from 5% to 30% by weight of the pediatric formula (i.e., the dry weight). The carbohydrate component is typically present in an amount of from 40% to 75% by weight of the pediatric formula, (i.e., the dry weight). The fat component is typically present in an amount of from 10% to 25% by weight of the pediatric formula, (i.e., the dry weight). Alternatively, the composition of the invention is a product aimed at adults, i.e. an adult formula. As used herein, the terms "adult formula" and "adult nutritional product" as used herein are used interchangeably to refer to nutritional compositions suitable for generally maintaining or improving the health of an adult. When the nutritional powder is formulated as an adult nutritional product, the protein component is typically present in an amount of from 5% to 35% by weight of the adult nutritional product, including from 10% to 25%, and including from 20% to 30% by weight of the adult nutritional product (dry weight). The carbohydrate component is typically present in an amount of from 40% to 80% by weight of the adult nutritional product, including from 50% to 75%, and also including from 60% to 75% by weight of the adult nutritional product. The fat component is typically present in an amount of from 0.5% to 20%, including from 1% to 15%, and also including from 15% to 20% by weight of the adult nutritional product.

Generally, any source of protein may be used so long as it is suitable for oral nutritional compositions and is otherwise compatible with any other selected ingredients or features in the nutritional composition. Non-limiting examples of suitable proteins (and sources thereof) suitable for use in the nutritional powders described herein include, but are not limited to, intact, hydrolyzed, or partially hydrolyzed protein, which may be derived from any known or otherwise suitable source such as milk (e.g., casein, whey), animal (e.g., meat, fish), cereal (e.g., rice, corn, wheat), vegetable (e.g., soy, pea, potato, bean), and combinations thereof. The protein may also include a mixture of amino acids (often described as free amino acids) known for use in nutritional products or a combination of such amino acids with the intact, hydrolyzed, or partially hydrolyzed proteins described herein. The amino acids may be naturally occurring or synthetic amino acids.

More particular examples of suitable protein (or sources thereof) used in the nutritional powders disclosed herein include, but are not limited to, whole cow's milk, partially or completely defatted milk, milk protein concentrates, milk protein isolates, nonfat dry milk, condensed skim milk, whey protein concentrates, whey protein isolates, acid caseins, sodium caseinates, calcium caseinates, potassium caseinates, legume protein, soy protein concentrates, soy protein isolates, pea protein concentrates, pea protein isolates, collagen proteins, potato proteins, rice proteins, wheat proteins, canola proteins, quinoa, insect proteins, earthworm proteins, fungal (e.g., mushroom) proteins, hydrolyzed yeast, gelatin, bovine colostrum, human colostrum, glycol macropeptides, mycoproteins, proteins expressed by microorganisms (e.g., bacteria and algae), and combinations thereof. The nutritional powders described herein may include any individual source of protein or combination of the various sources of protein listed above. In addition, the proteins for use herein can also include, or be entirely or partially replaced by, free amino acids known for use in nutritional products, non-limiting examples of which include L-tryptophan, L-glutamine, L-tyrosine, L-methionine, L-cysteine, taurine, L-arginine, L-carnitine, and combinations thereof.

The carbohydrate or source of carbohydrate suitable for use in the composition disclosed herein may be simple, complex, or variations or combinations thereof. Generally, the carbohydrate may include any carbohydrate or carbohydrate source that is suitable for use in oral nutritional compositions and is otherwise compatible with any other selected ingredients or features in the nutritional powder. Non-limiting examples of carbohydrates suitable for use in the nutritional powders described herein include, but are not limited to, polydextrose, maltodextrin; hydrolyzed or modified starch or cornstarch; glucose polymers; corn syrup; corn syrup solids; sucrose; glucose; fructose; lactose; high fructose corn syrup; honey; sugar alcohols (e.g., maltitol, erythritol, sorbitol); isomaltulose; sucromalt; pullulan; potato starch; and other slowly-digested carbohydrates; dietary fibers including, but not limited to, fructooligosaccharides (FOS), galactooligosaccharides (GOS), oat fiber, soy fiber, gum arabic, sodium carboxymethylcellulose, methylcellulose, guar gum, gellan gum, locust bean gum, konjac flour, hydroxypropyl methylcellulose, tragacanth gum, karaya gum, gum acacia, chitosan, arabinogalactans, glucomannan, xanthan gum, alginate, pectin, low methoxy pectin, high methoxy pectin, cereal beta-glucans (e.g., oat beta-glucan, barley beta-glucan), carrageenan and psyllium, soluble and insoluble fibers derived from fruits or vegetables; other resistant starches; and combinations thereof. The nutritional powders described herein may include any individual source of carbohydrate or combination of the various sources of carbohydrate listed above.

The fat or source of fat suitable for use in the nutritional powders described herein may be derived from various sources including, but not limited to, plants, animals, and combinations thereof. Generally, the fat may include any fat or fat source that is suitable for use in oral nutritional compositions and is otherwise compatible with any other selected ingredients or features in the nutritional powder. Non-limiting examples of suitable fat (or sources thereof) for use in the nutritional powders disclosed herein include coconut oil, fractionated coconut oil, soy oil, high oleic soy oil, corn oil, olive oil, safflower oil, high oleic safflower oil, medium chain triglyceride oil (MCT oil), high gamma linolenic (GLA) safflower oil, sunflower oil, high oleic sunflower oil, palm oil, palm kernel oil, palm olein, canola oil, high oleic canola oil, marine oils, fish oils, algal oils, borage oil, cottonseed oil, fungal oils, eicosapentaenoic acid (EPA), docosahexaenoic acid (DHA), arachidonic acid (ARA), conjugated linoleic acid (CLA), alpha-linolenic acid, rice bran oil, wheat bran oil, interesterified oils, transesterified oils, structured lipids, and combinations thereof. Generally, the fats used in nutritional powders for formulating infant formulas and pediatric formulas provide fatty acids needed both as an energy source and for the healthy development of the infant, toddler, or child. These fats typically comprise triglycerides, although the fats may also comprise diglycerides, monoglycerides, and free fatty acids. Fatty acids provided by the fats in the nutritional powder include, but are not limited to, capric acid, lauric acid, myristic acid, palmitic acid, palmitoleic acid, stearic acid, oleic acid, linoleic acid, alpha-linolenic acid, ARA, EPA, and DHA. The nutritional powders can include any individual source of fat or combination of the various sources of fat listed above. Preferably, the fat is a mixture of vegetable fat and milk fat such as obtained from milk from a mammal like cow, sheep, goat, mare, or camel. More preferably, wherein the milk fat is bovine milk fat. Mixtures of different types of fat are preferred because they help to provide different fatty acids and better resemble the type of linkage between the glycerol moiety and the fatty acid moiety in the fat, when compared to human mother's milk.

In certain embodiments, the composition described herein may further comprise other optional ingredients that may modify the physical, chemical, hedonic, or processing characteristics of the products or serve as additional nutritional components when used for a targeted population. Many such optional ingredients are known or otherwise suitable for use in other nutritional products and may also be used in the nutritional powders described herein, provided that such optional ingredients are safe and effective for oral administration and are compatible with the essential and other ingredients in the selected product form. Non-limiting examples of such optional ingredients include preservatives, antioxidants, emulsifying agents, buffers, additional nutrients as described herein, colorants, flavors (natural, artificial, or both), thickening agents, flow agents, anti-caking agents, and stabilizers.

In certain embodiments, the composition further comprise minerals, non-limiting examples of which include calcium, phosphorus, magnesium, iron, zinc, manganese, copper, sodium, potassium, molybdenum, chromium, selenium, chloride, and combinations thereof.

In certain embodiments, the composition further comprise vitamins or related nutrients, non-limiting examples of which include vitamin A, vitamin D, vitamin E, vitamin K, thiamine, riboflavin, pyridoxine, vitamin B12, niacin, folic acid, pantothenic acid, biotin, vitamin C, choline, inositol, salts and derivatives thereof, and combinations thereof.

In another embodiment, the composition for use of the invention, further comprises one or more dietary fibers, preferably wherein the dietary fiber is a prebiotic dietary fiber. In yet another embodiment, it further comprises one or more prebiotic dietary fibers wherein the prebiotic dietary fiber is selected from one or more of the group consisting of GOS, FOS, inulin and resistant starch, preferably GOS, FOS and inulin, particularly it further comprises one or more of the group consisting of FOS, inulin and resistant starch. The composition of the invention may further comprise one or more probiotics. Probiotics and prebiotics are known in the art and are claimed to have beneficial effect on the subject's gut microbiome and to have a positive effect on the subject's health and / or wellbeing. Probiotics are live microorganisms promoted with claims that they provide health benefits when consumed, generally by improving or restoring the gut flora. Probiotics are well known in the art and examples include *Saccharomyces boulardii* (a yeast) and bacteria in the Lactobacillus and Bifobacterium families of microorganisms. *Lactobacillus acidophilus* is the probiotic that is found in some yogurts. Preferably, the probiotic is a probiotic known to be effective on the respiratory tract, e.g. as described in Tapiovaara *et al,* (Tapiovaara et al, Pediatric Infectious diseases 2016 Vol 1 No3:19, http://dx.doi.org/10.21767/2573-0282.100019).

In one embodiment, in the combination of 2'-FL and prebiotic dietary fibers in relation to the invention, the weight ratio between 2'-FL and the dietary fiber is in the range of from 0.5:10 to 10:0.5. Preferably, the composition comprises at least 5 wt.% of 2'-FL and at least 5 wt% of dietary fiber as compared to the total dry weight of the composition, more preferably it comprises at least 10 wt% 2'FL and at least 10 wt% dietary fiber, even more preferably, it comprises at least 20 wt% 2'FL and at least 20 wt% dietary fiber.

It is believed that the combination of 2'-FL and FOS, inulin and/or resistant starch in relation to the invention provides better results as it allows more 2'-FL to arrive in the distal part of the colon. In one embodiment, the weight ratio between 2'-FL and FOS, inulin and/or resistant starch in the composition is in the range of from 0.5:10 to 10:0.5. In one embodiment, the weight ratio between 2'-FL and inulin in the composition is in the range of from 0.5:10 to 10:0.5. In one embodiment, the weight ratio between 2'-FL and FOS in the composition is in the range of from 0.5:10 to 10:0.5.

In one embodiment, when the composition of the invention is comprising 2'-FL and dietary fiber, these are preferably present in an amount of at least 0.1 wt. % of 2'-FL and 1 wt.% of dietary fiber as compared to the total dry weight of the composition. In one embodiment the composition comprises at least 0.01 wt.% of 2'-FL and at least 5 wt.% of dietary fiber as compared to the total dry weight of the composition. In one embodiment the composition comprises at least 0.1 wt.% of 2'-FL and at least 5 wt.% of dietary fiber as compared to the total dry weight of the composition. In another embodiment it comprises at least 5 wt.% of 2'-FL and 5 wt.% of dietary fiber as compared to the total dry weight of the composition. In yet another embodiment, 2'-FL is present in an amount of at least at least 0.01 wt%, e.g. at least 0.1 wt%, at least 1 wt%, or at least 10 wt.% as compared to the total dry weight of the composition, preferably at least 20 wt.%, at least 30 wt.%, at least 40 wt.%, at least 50 wt.%, at least 60 wt.%, at least 70 wt.%, at least 80 wt.%, at least 90 wt.%, e.g. up to 91 wt%, 92 wt%, 93 wt%, or 94 wt% as compared to the total dry weight of the composition.

The composition comprising 2'-FL and dietary fiber may, in one embodiment, be a kit of parts wherein the 2'-FL and dietary fiber are not mixed in a single composition, i.e. 2'-FL and dietary fiber are comprised in two separate compositions, when taken together with optional other components, these separate compositions create the composition of the invention. As will be understood by those skilled in the art, the benefits of the present invention may be obtained by sequential or simultaneous administration of 2'-FL and dietary fiber. Such uses and methods of treatment are also within the scope of the present invention. Preferably, 2'-FL and dietary fiber are administered simultaneously or sequentially within less than 10 minutes, more preferably less than 5 minutes, most preferably less than 1 minute.

For most humans, there is a maximum in the total amount of dietary fiber, including 2'FL that can be consumed on a daily basis, an amount above which gastro-intestinal discomfort starts to develop. The maximum amount of dietary fiber that can be consumed per day may be determined by gradually increasing the daily amount of dietary fiber and monitoring if adverse effects occur, one may for example start with 1 gram of 2'FL per day and increase with 3 grams per week. For 2'FL a daily dosage of 20 g has been tested as safe. It is known that fermentation of GOS does not result in an increase of acetate levels (i.e. acetate concentration) in the distal colon, i.e. the descending colon (the left side of the colon) and the sigmoid colon (the S-shaped section of the colon that connects to the rectum) (Canfora et al. Gastroenterology, 2017, vol 153 No. 1 pp 87-97). So in another embodiment the composition comprising 2'FL does not comprise galacto-oligosaccharides (GOS), as that allows for a higher amount of 2'FL to be consumed without gastro-intestinal discomfort. In this embodiment, the term GOS does not include lactose. In another embodiment, the composition of the invention comprising 2'FL does not comprise GOS and does not comprise lactose.

In still another embodiment, the composition for use of the invention comprising 2'FL is in the form of single serving or a multitude of single servings. Such single servings may be individually packed.

In one embodiment the amount of 2'-FL per daily serving in the composition as used in the invention is more than 0.01 gram preferably more than 0.1 gram. In another embodiment, the amount of 2'FL per daily serving is in the range of from 0.01 to 30 gram per daily serving, preferably it is in the range of from 0.1 to 30 gram per daily serving, more preferably in a range of from 0.5 to 25 gram, even more preferably in a range of from 1 to 20 gram, most preferably in a range of from 1 to 10 gram per daily serving. In still another embodiment, the amount of 2'FL is between 0.01 and 1.0 gram per daily serving.

In another embodiment the amount of 2'FL in the composition for use in the invention is at least 0.1 wt% compared to the total dry weight of the composition, such as at least 0.5 wt, or at least 1 wt%. In one embodiment it is at least 5wt% compared to the total dry weight of the composition, preferably at least 10 wt%, more preferably at least 15wt% even more preferably at least 20 wt%, particularly preferably at least 30wt%, most preferably at least 40 wt%.

In another embodiment, the composition for use of the invention comprises 2'-FL and dietary fiber, wherein i) the amount of 2'-FL per daily serving is greater than 0.01 g and ii) the total amount of 2'-FL and dietary fiber per daily serving is less than 30 gram, preferably less than 25 gram, more preferably less than 20 gram, most preferably less than 15 gram.

In another embodiment, the composition for use of the invention comprises 2'- FL and dietary fiber, wherein i) the amount of 2'-FL per daily serving is greater than 0.1 g and ii) the total amount of 2'-FL and dietary fiber per daily serving is less than 30 gram, preferably less than 25 gram, more preferably less than 20 gram, most preferably less than 15 gram. In yet another embodiment, the composition for use of the invention comprises 2'- FL and dietary fiber, wherein the amount of 2'-FL per daily serving is between 0.1 and 8 gram and the amount of dietary fiber per daily serving is between 1 and 6 grams.

The prevention against RNA virus infection and / or treatment of RNA virus infection as herein described can be characterized by a reduced viral load in subjects. Preferably the viral load is tested using lab-testing, e.g. such as available from https://labtestsonline.org e.g. under /tests/respiratory-syncytial-virus-rsv-testing or from https://www.fishersci.nl/ under shop/products/oxoid-imagen-respiratory-virus-screen/13275519; or as described by Sarna et al (Timing of First Respiratory Virus Detections in Infants: A Community-Based Birth Cohort Study JID 2018:217 (1 February) pp 418-427, or alternatively as described by Duarte de Souza et al, J Bras Pneumol 2016;42(4):261-265, Methods https://doi.org/10.1590/s1806-37562015000000241.

Briefly, such laboratory testing using a PCR assay, consists of the following steps:
i. collection of an anterior nose swab or a nasopharyngeal lavage;
ii. submitting samples to PCR for RSV or other viruses: extracting total RNA, production of cDNA and quantification. Quantitative PCR reactions are performed to amplify RSV-, or other virus-specific genes using cDNA in triplicate for each subject. For example by using previously validated real-time polymerase chain reaction (PCR) assays [Sarna M, Alsaleh A, Lambert SB, et al. Respiratory viruses in neonates: a prospective, community-based birth cohort study. Pediatr Infect Dis J 2016; 35:1355-7; Lambert SB, Ware R, Cook A, et al. Observational Research in Childhood Infectious Diseases (ORChID): a dynamic birth cohort study. BMJ Open 2012; 2:e002134-40; Alsaleh AN, Whiley DM, Bialasiewicz S, et al. Nasal swab samples and real-time polymerase chain reaction assays in community-based, longitudinal studies of respiratory viruses: the importance of sample integrity and quality control. BMC Infect Dis 2014; 14:15]; Generate a standard curve.

The viral load (in copies/mL) is calculated from the amount of cDNA used in the PCR. Statistical analysis of the viral load is performed e.g. using GraphPad Prism, version 5.02 (Graphpad Software, San Diego, CA, USA). This test allows for a determination of virus type and quantification.

Alternatively, the amount of viral RNA may be determined in the bronchoalveolar lavage fluid (BALF) using methods known in the art.

Alternatively, the prevention and /or treatment may be determined by recording daily symptoms of the infection on a diary card, which listed predefined respiratory symptoms (nasal congestion or discharge, dry or wet [moist] cough, pneumonia, ear infection [acute otitis media], rattly breathing, shortness of breath, and wheezing). In case the virus is a non-respiratory virus, symptoms relevant for that specific virus should be recorded.

Symptoms recorded for a healthy, non-infected person should be taken as the negative reference. Symptoms recorded for an infected person, virus infection diagnosed and confirmed by a doctor e.g. GP, as a negative control. The average scores of at least ten persons per category. In this way the scores for persons infected with the virus and taking the composition of the invention, should be compared with the scores infected and not taking the composition of the invention. The virus load is reduced by at least 10% by using Ct scores as compared to persons not taking the composition of the invention, preferably, by at least 20%, such as at least 30%, 40%, 50%, 60%, 70%, 80% or even at least 90% reduction.

In one embodiment, the reduced virus load is determined by extracting virus nucleic acids from a sample of the subjects respiratory tract wherein the nucleic acid is a ribonucleic acid, preferably wherein the sample of the subjects respiratory tract is a nasopharyngeal lavage.

The composition for use according to the invention is a food product, preferably selected from the group consisting of dairy product e.g. as milk-product, milkshake, chocolate milk, yoghurt, cream, cheese, pudding, ice cream etc ; bar, such as nutritional bar, energy bar, snack bar, cereal bar, bar for diabetics etc.; liquid product, such as nutritional drink, diet drink, liquid meal replacers, sports drink and other fortified beverages; savory snack, such as chips, tortillas, puffed and baked snacks, crackers, pretzels; savory biscuit, bakery products, such as muffins, cakes, biscuits; pasta, such as spaghetti; and food supplement e.g. pills, capsules, or dry powder. Food supplements may be ready for consumption or may need to be dissolved in a liquid like water. The product in dry powder form may be accompanied with a device, such as a spoon, to measure the desired amount of the powder (e.g. daily or unit dose). Food supplements may further comprise other ingredients commonly used in food supplements such as vitamins, minerals, salts, etc. The food product is preferably selected from the group consisting of dairy product, liquid product, and food supplement. Alternatively, the composition is a food supplement such as a pill or powder, preferably a pill. The food product preferably is accompanied with instructions specifying how much of the product should be consumed on a daily basis.

The composition as defined herein or the nutritional composition as defined herein may be provided in a jar, bottle, sachet, carton, wrapping, and the like.

Typically, the treatment entails the administration of the 2'-FL and optional further ingredients in unit dose form. In one embodiment the treatment entails administering a composition according to the invention in a unit dose form. In a preferred embodiment, the composition is provided in the form of a single serving, optionally each single serving is individually packaged, and each serving comprising the 2'-FL. Alternatively, the composition for use according to the invention is present in a container comprising a multitude of single servings such as 5, 7, 10, 30, 50, 100, 200, 300, 365 single servings. Such a container preferably is accompanied with instructions on the amount of the composition representing a single serving or daily serving. In still another embodiment, the container comprising the composition of the invention comprises at least 7, for example at least 14 or at least 31 single servings.

In one embodiment, the unit dose amount of the 2'-FL is at least 0.01 gram, preferably at least 0.1 gram, 1 gram, e.g. at least 1.5 gram, at least 2 gram, at least 2.5 gram, at least 3 gram, at least 3.5 gram, or at least 4 gram.

In another embodiment, the unit dose amount of the 2'-FL is at most 30 grams, e.g. at most 25 grams, such as at most 20 grams, at most 15 grams, at most 12.5 grams, at most 10 grams, at most 9 grams, at most 8 grams, at most 7 grams, at most 6 g or at most 5 grams.

In one embodiment, the unit dose amount of the dietary fiber is 0.5-10 grams and the unit dose amount of the 2'-FL is 3-8 grams, preferably 4-8 grams, more preferably 5-8 grams 2'-FL. In another embodiment, the unit dose amount of the dietary fiber is 2-8 grams and the unit dose amount of the 2'-FL is 0.5-10 grams, preferably 1- 7 grams, more preferably 2-4 grams of 2'-FL. In still another embodiment, the unit dose amount of the dietary fiber is at least 1 grams, at least 1.5 grams, at least 2 grams, at least 2.5 grams, at least 3 grams, at least 3.5 grams, or at least 4 grams. In another embodiment, the unit dose amount of the dietary fiber is at most 25 grams, at most 20 grams, at most 15 grams, at most 12.5 grams, at most 10 grams, at most 9 grams, at most 8 grams, at most 7 grams, at most 6 g or at most 5 grams. Preferably the dietary fiber is selected from one or more of the group consisting of GOS, FOS, inulin, and resistant starch, more preferably from one or more of the group consisting of FOS and inulin.

Servings or doses of the composition for use of the invention are preferably administered at least once a week, more preferably at least once every 3 days, even more preferably at least once every other day, most preferably at least once daily. In one embodiment of the invention, the treatment comprises the daily administration of unit doses of the composition of the invention. It is believed that the daily administration of the composition of the invention results in a more continuous production of acetate in the distal colon which provides protection against viral infection.

In accordance with the invention, the use and/ or treatment as defined herein before, is preferably continued for a period of at least two weeks, e.g. at least 3 weeks, at least 4 weeks, at least 1 month, at least two months, at least three months, at least 4 months, at least 5 months, or even at least 6 months.

In another embodiment of the invention, the use and/or treatment comprises the daily administration of a composition comprising 2'FL in an amount of between 0.01 and 5 g and optionally dietary fiber in an amount of 2 to 24 g.

A method of preventing and/or treating a human subject from becoming infected with an RNA virus infection by providing the subject an nutritional composition comprising 2'FL wherein the composition and virus are as herein defined above is disclosed.

The use of a composition comprising 2'FL (i.e. a composition as herein defined above) to prevent or treat an RNA viral infection in a human subject characterized in that the composition is administered to the subject wherein the composition, daily dosage and RNA virus are as defined herein above, is disclosed.

Further it is disclosed a method for treating and/or preventing RNA - viral infection in a human subject e.g. an infant, toddler, child, adult or elderly person; the method comprising administering to the subject a composition comprising 2'FL, preferably from 0.001 mg to 20 g of 2'-FL, more preferably from 0.1 gram to 10 gram, most preferably from 0.4 gram to 5 gram per daily serving.

In a particular embodiment of the invention, the composition for use is presented in the form of individually packaged single servings. The term "single serving" as used herein refers to a certain quantity and/or size of the product that is adequate for consumption as a single portion for a single person. Such products may be in a form that is ready-to-eat or ready-to-consume or it may be in a form that requires further processing, such as heating or addition of a quantity of hot or cold water. In one embodiment, the composition of the invention is presented in the form of individually packaged single servings, wherein each serving contains a unit dose as defined herein elsewhere.

A preferred product form of the composition used in the invention is an edible bar, such as a nutritional bar, energy bar, diet bar or food supplement bar, snack bar, etc., examples of which are well known to those of skill in the art. The edible bar may in one embodiment be a cereal composition comprising a cereal mix and a binding syrup. The binding syrup can include e.g. glucose syrup, granulated sugar, glycerol, water, emulsifier, fat and flavors. The dietary fiber composition of the invention can suitably be incorporated in the binding syrup. Products of this type are known by those skilled in the art, e.g. from international patent publication no. WO 2017/078519.

Practice within the numerical limits stated, is generally preferred. Also, unless expressly stated to the contrary: percent, "parts of," and ratio values are by weight; the description of a group or class of materials as suitable or preferred for a given purpose in connection with the invention implies that mixtures of any two or more of the members of the group or class are equally suitable or preferred; description of constituents in chemical terms refers to the constituents at the time of addition to any combination specified in the description, and does not necessarily preclude chemical interactions among the constituents of a mixture once mixed; the first definition of an acronym or other abbreviation applies to all subsequent uses herein of the same abbreviation and applies, *mutatis mutandis*, to normal grammatical variations of the initially defined abbreviation; and, unless expressly stated to the contrary, measurement of a property is determined by the same technique as previously or later referenced for the same property.

The invention is hereinafter illustrated with reference to the following, non-limiting, examples.

### EXAMPLES

### In vitro fermentation model, TIM-2

The *in* vitro fermentation studies were done using the TIM-2 model. This is a validated, dynamic, computer-controlled model that simulates the human colon, mimicking body temperature, lumen pH, absorption of water and microbial metabolites through a semipermeable membrane inside the model, mixing and transporting the intestinal contents with peristaltic movements, using an anaerobic microbiota from human origin, it corresponds basically to the model as described in Minekus, M., et al. Appl. Microbiol. Biotechnol. 1999 53, 108-114. doi: 10.1007/ s002530051622 and Kortman et al., Frontiers in Microbiology 2016, 6, 1481. Characteristics of the movements of the contents in the TIM-2 system was simulated using an increase of the pH and peristaltic movements of the contents in the system using peristaltic pumps as described in Minekus, M (1998. Development and validation of a dynamic model of the gastrointestinal tract. PhD thesis, Delft University of Technology, The Netherlands).

### SCFA analysis

SCFA analysis was performed at Brightlabs B.V., Venlo, The Netherlands), according to (Sáyago-Ayerdi SG, et al. Food Research International, E-pub date 13 December 2017; Sayago Ayerdi et al Food Research International 118 (2019) 89-95).

### Inoculum

Pooled fecal microbiota samples from 20 healthy subjects with a BMI ≥18.5 kg/m² (including both lean (18.5 kg/m² ≤ BMI < 25 kg/m²) and obese (BMI ≥ 30 kg/m²) subjects) not suffering from any metabolic disease was used to inoculated the TIM-2 *in vitro* fermentation model.

### Vitamine mixture

A vitamin mixture was used containing (per liter): 1 mg menadione, 2 mg D-biotin, 0.5 mg vitamin B12, 10 mg pantothenate, 5 mg nicotinamide, 5 mg p-aminobenzoic acid and 4 mg thiamine.

### Dialysate

The dialysate used in the TIM-2 system contained (per liter): 2.5 g K₂HPO₄·3H₂O, 4.5 g NaCl, 0.005 g FeSO₄·7H₂O, 0.5 g MgSO₄·7H₂O, 0.45 g CaCl₂·2H₂O, 0.05 g bile and 0.4 g cysteine·HCl, plus 1 mL of the vitamin mixture.

### Example 1

The fecal microbiota was freshly sampled in and stored directly (within 2h) on ice and under anaerobic conditions. Next, in an anaerobic cabinet, samples were diluted 1:1 with dialysate, and pooled at approximately equal weight, after which glycerol was added (to a final concentration of 12-13 w/w) and aliquots (30 ml/tube) were frozen in liquid nitrogen and stored at -80 °C.

Prior to inoculation, 4x 30-ml aliquots were taken from the -80 °C freezer and thawed in a water bath at 37°C for exactly 1 hour (still under anaerobic conditions). In an anaerobic cabinet, the microbiota from the 4 tubes was combined and the same volume of pre-reduced (i.e. oxygen free) dialysate was added, gently mixed and divided over 4 syringes each comprising ca. 60 ml of microbiota-containing liquid. The syringes were sealed with a small flexible tube closed with a tubing clamp. Each TIM-2 unit was inoculated with 1 of the 4 syringes (i.e. 60 ml microbiota/dialysate mixture), using one single sample port to inoculate a TIM-2 unit. After the microbiota was introduced into the unit, another 60 ml of pre-reduced dialysate was added into the TIM-2 unit to get to a final volume of 120 ml per unit (i.e. system).

To simulate the conditions in the proximal region of the colon, the colon transversum and the distal part of colon, the pH of the microbiota/dialysate mixture was increased from pH 5.8 to pH 7.0 using 1M NaOH over a period of 24 hours. The increase in pH simulated the movement of fibers through the colon during the 24 hours' experiment (wherein the last 16 hours simulated the more distal colonic site (i.e. transverse + distal)).

Samples (1 mL) were taken for Short Chain Fatty Acid (SCFA) analysis after 1, 2, 4, 6, 8 and 24 h after test product insertion; cumulative absolute amounts of SCFA were determined.

Samples were centrifuged at 14,000 rpm for 10 min, filtered through a 0.45 µm PFTE filter, and diluted in the mobile phase (1.5mM aqueous sulfuric acid). Ten microliters were loaded into the column with the help of an automatic sampler 730 (Metrohm, Herisa, Switzerland). The acids were eluted according to their pKa. The analysis was carried out by ion exclusion chromatography (IEC) using an 883 chromatograph (IC, Metrohm) equipped with a Transgenomic IC Sep ICE-ION-300 column (30 cm×7.8mm×7 µm) and a MetroSep RP2 Guard. A column flow of 0.4 mL/min with a column temperature of 65 °C was used. The acids were detected using suppressed conductivity detection. Analyses were performed by Brightlabs (Venlo, The Netherlands).

### Addition of the test-product:

After an adaption period of 40h, 7.5 grams of 2'-fucosyllactose (2'-FL) was introduced into a TIM-2 unit through the sample port (on Wednesday) as a single shot.

An experimental week contained the following steps:
Monday: Start up all 4 units of the TIM-2 system (pH 5.8).
Tuesday: Feeding of Simulated ileal efflux environment medium (SIEM) (Maathuis et al 2009 Journal of the American College of Nutrition 28(6):657-66 DOI: 10.1080/ 07315724.2009.10719798);
Simulated ileal efflux medium (SIEM) contained 5.7 g/liter BD Bacto tryptone (BD), 2.4 g/liter D-glucose (Sigma-Aldrich), 6.14 g/liter NaCl (Roth, Germany), 0.68 g/liter KH₂PO₄ (Merck, Germany), 0.3 g/liter NaH₂PO₄ (Merck, Germany), 1.01 g/liter NaHCO₃ (Merck, Germany), 5.6 g/liter bile salts no. 3 (Difco), 0.2 g/liter lysozyme (Serva, Germany), 1,000 U α-amylase (Fluka, Germany), 110 U trypsin (Sigma-Aldrich), 380 U chymotrypsin (Calbiochem, Germany), and 960 U lipase (Sigma-Aldrich). D(+)-Glucose and enzymes were filter sterilized before addition.
Wednesday: 3 h starvation period followed by a single insertion of test product; 2'-FL (7.5 gram of 2'-FL) was added through the sample port]; following introduction of the test product, samples for SCFA analysis were taken after 1, 2, 4, 6 and 8 h.
Thursday: 24 h after insertion of the test product: last sample was taken for SCFA analysis;
Friday: cleaning (the experiment was executed in one week).

### Results

The increase in cumulative absolute acetate amount between the last two sampling points (i.e. between 8 and 24 h after product insertion) was taken as an indication for the increase in SCFA and of acetate in the distal colon.

The results of the experiments are displayed below in Table 1.

**Table 1. Results of the TIM-2 experiments, using microbiota samples subjects to which 2'-FL was administered.**

| **Test product** | **Acetate production *** |
|---|---|
| 2'-FL | 17 mmol |

| | |
|---|---|
| * the amount of acetate refers to the amounts produced in between 8 and 24 hours after insertion of the test product; representing the amounts of acetate and SCFA produced in the distal colon. | |

The experiment shows that insertion of 2'FL (7.5 gram) in the model system results in an increased acetate production of 17 mmol acetate in the distal colon. An increase in the acetate production was observed both when fecal microbiota samples of lean and obese subjects was used in the TIM-2 model system. An increase of acetate in the distal colon results in higher levels of acetate in the circulation, which will give the claimed effect.

## Claims

1. A composition comprising 2'-fucosyllactose (2'-FL) for use in the prevention against and / or the treatment of RNA-virus infection by increasing the production of short chain fatty acids (SCFA) in the distal colon; the composition is a nutritional composition and wherein the virus is a Group IV type virus as defined in the Baltimore Classification of viruses.

2. The composition for use of claim 1, wherein the composition is a food product, preferably wherein the food product is selected from the group consisting of dairy product, bar, liquid product, savory snack, savory biscuit, bakery products, pasta and food supplement.

3. The composition for use of any of the preceding claims, further comprising one or more probiotics.

4. The composition for use of any of the preceding claims, further comprising one or more dietary fibers, preferably wherein the dietary fiber is a prebiotic dietary fiber, more preferably wherein the prebiotic dietary fiber is selected from the group consisting of galactooligosaccharides (GOS), fructooligosaccharides (FOS), inulin and resistant starch.

5. The composition for use of claim 4, wherein the weight ratio between 2'-FL and the dietary fiber is in the range of from 0.5:10 to 10:0.5.

6. The composition for use of claim 4 or 5, wherein the composition comprises at least 0.01 wt.% of 2'-FL and at least 5 wt.% of dietary fiber as compared to the total dry weight of the composition, preferably wherein the composition comprises at least 5 wt.% of 2'-FL and at least 5 wt.% of dietary fiber as compared to the total dry weight of the composition.

7. The composition for use of any of the preceding claims, wherein the composition does not comprise galacto-oligosaccharides (GOS).

8. The composition for use of any one of the preceding claims, wherein the composition is in the form of single serving; or a multitude of single serving, preferably, the single servings are individually packaged.

9. The composition for use of claim 8, wherein the amount of 2'-FL per daily serving is in the range of from 0.01 to 30 grams, preferably in the range of from 0.1 to 30 grams, more preferably in a range of from 0.5 to 25 gram, particularly preferably in a range of from 1 to 20 gram, most preferably in a range of from 1 to 10 gram.

10. The composition for use of claim 9, wherein
i. the amount of 2'-FL per daily serving is greater than 0.01 g and
ii) the total amount of 2'-FL and dietary fiber per daily serving is less than 30 gram, preferably less than 25 gram, more preferably less than 20 gram, most preferably less than 15 gram.

## Patentansprüche

1. Zusammensetzung, umfassend 2'-Fucosyllactose (2'-FL) zur Verwendung bei der Vorbeugung und/oder der Behandlung von RNA-Virus-Infektionen durch das Erhöhen der Produktion von kurzkettigen Fettsäuren (SCFA= short chain fatty acids) im distalen Dickdarm; wobei es sich bei der Zusammensetzung um eine Nahrungszusammensetzung handelt und wobei es sich bei dem Virus um ein in der Baltimore-Klassifizierung von Viren als Virus des Typs der Gruppe IV definiertes handelt.

2. Zusammensetzung zur Verwendung nach Anspruch 1, wobei es sich bei der Zusammensetzung um ein Nahrungsmittelprodukt handelt, vorzugsweise wobei das Nahrungsmittelprodukt aus der Gruppe bestehend aus Milchprodukt, Riegel, Flüssigprodukt, herzhaftem Snack, herzhaftem Keks, Bäckereiprodukten, Pasta und Nahrungsergänzungsmittel ausgewählt ist.

3. Zusammensetzung zur Verwendung nach einem der vorhergehenden Ansprüche, ferner umfassend ein oder mehrere Probiotika.

4. Zusammensetzung zur Verwendung nach einem der vorhergehenden Ansprüche, ferner umfassend ein oder mehrere Ballaststoffe, vorzugsweise wobei es sich bei dem Ballaststoff um einen präbiotischen Ballaststoff handelt, weiter bevorzugt wobei der präbiotische Ballaststoff aus der Gruppe bestehend aus Galacto-Oligosacchariden (GOS), Fructo-Oligosacchariden (FOS), Inulin und resistenter Stärke ausgewählt ist.

5. Zusammensetzung zur Verwendung nach Anspruch 4, wobei das Gewichtsverhältnis zwischen 2'-FL und dem Ballaststoff im Bereich von 0,5:10 bis 10:0,5 liegt.

6. Zusammensetzung zur Verwendung nach Anspruch 4 oder 5, wobei die Zusammensetzung mindestens 0,01 Gew.-% 2'-FL und mindestens 5 Gew.-% Ballaststoff im Vergleich zum Gesamttrockengewicht der Zusammensetzung umfasst, vorzugsweise wobei die Zusammensetzung mindestens 5 Gew.-% 2'-FL und mindestens 5 Gew.-% Ballaststoff im Vergleich zum Gesamttrockengewicht der Zusammensetzung umfasst.

7. Zusammensetzung zur Verwendung nach einem der vorhergehenden Ansprüche, wobei die Zusammensetzung keine Galacto-Oligosaccharide (GOS) umfasst.

8. Zusammensetzung nach einem der vorhergehenden Ansprüche, wobei die Zusammensetzung in Form von einer Einzelportion oder mehreren Einzelportionen vorliegt, vorzugsweise wobei die Einzelportionen individuell verpackt sind.

9. Zusammensetzung zur Verwendung nach Anspruch 8, wobei die Menge von 2'-FL pro Tagesportion im Bereich von 0,01 bis 30 Gramm, vorzugsweise im Bereich von 0,1 bis 30 Gramm, weiter bevorzugt in einem Bereich von 0,5 bis 25 Gramm, insbesondere bevorzugt in einem Bereich von 1 bis 20 Gramm, am meisten bevorzugt in einem Bereich von 1 bis 10 Gramm liegt.

10. Zusammensetzung zur Verwendung nach Anspruch 9, wobei
i. die Menge von 2'-FL pro Tagesportion mehr als 0,01 g beträgt und
ii) die Gesamtmenge von 2'-FL und Ballaststoff pro Tagesportion weniger als 30 Gramm, vorzugsweise weniger als 25 Gramm, weiter bevorzugt weniger als 20 Gramm, am meisten bevorzugt weniger als 15 Gramm beträgt.

## Revendications

1. Composition comprenant du 2'-fucosyllactose (2'-FL) pour utilisation dans la prévention contre et/ou le traitement d'une infection par un virus à ARN en augmentant la production d'acides gras à chaîne courte (SCFA) dans le côlon distal ; la composition étant une composition nutritionnelle et dans laquelle le virus est un virus du groupe IV tel que défini dans la classification des virus de Baltimore.

2. Composition pour utilisation selon la revendication 1, dans laquelle la composition est un produit alimentaire, de préférence dans laquelle le produit alimentaire est choisi dans un groupe constitué d'un produit laitier, d'une barre, d'un produit liquide, d'une collation salée, d'un biscuit salé, de produits de boulangerie, de pâtes et de compléments alimentaires.

3. Composition pour utilisation selon l'une quelconque des revendications précédentes, comprenant en outre un ou plusieurs probiotiques.

4. Composition pour utilisation selon l'une quelconque des revendications précédentes, comprenant en outre une ou plusieurs fibres alimentaires, de préférence dans laquelle la fibre alimentaire est une fibre alimentaire prébiotique, plus préférablement dans laquelle la fibre alimentaire prébiotique est choisie dans un groupe constitué des galacto-oligosaccharides (GOS), des fructo-oligosaccharides (FOS), de l'inuline et de l'amidon résistant.

5. Composition pour utilisation selon la revendication 4, dans laquelle le rapport pondéral entre le 2'-FL et la fibre alimentaire est compris entre 0,5:10 et 10:0,5.

6. Composition pour utilisation selon la revendication 4 ou 5, dans laquelle la composition comprend au moins 0,01 % en poids de 2'-FL et au moins 5 % en poids de fibres alimentaires par rapport au poids sec total de la composition, de préférence dans laquelle la composition comprend au moins 5 % en poids de 2'-FL et au moins 5 % en poids de fibres alimentaires par rapport au poids sec total de la composition.

7. Composition pour utilisation selon l'une quelconque des revendications précédentes, dans laquelle la composition ne comprend pas de galacto-oligosaccharides (GOS).

8. Composition pour utilisation selon l'une quelconque des revendications précédentes, dans laquelle la composition est sous la forme d'une portion individuelle ; ou d'une multitude de portions individuelles, de préférence, les portions individuelles étant emballées individuellement.

9. Composition pour utilisation selon la revendication 8, dans laquelle la quantité de 2'-FL par portion quotidienne est comprise entre 0,01 et 30 grammes, de préférence entre 0,1 et 30 grammes, plus préférablement entre 0,5 et 25 grammes, particulièrement préférablement entre 1 et 20 grammes, le plus préférablement entre 1 et 10 grammes.

10. Composition pour utilisation selon la revendication 9, dans laquelle
i. la quantité de 2'-FL par portion quotidienne est supérieure à 0,01 g et
ii) la quantité totale de 2'-FL et de fibres alimentaires par portion quotidienne est inférieure à 30 grammes, de préférence inférieure à 25 grammes, plus préférablement inférieure à 20 grammes, le plus préférablement inférieure à 15 grammes.
